# EUROPEAN PATENT APPLICATION

(11) **EP 1 792 599 A1**
(43) Date of publication of application: **06.06.2007**
(21) Application number: 05785798.9
(22) Date of filing: 15.09.2005
(51) Int. Cl.: A61K 6/00

(54) **WHITENING AGENT**

(30) Priority: 24.09.2004 JP 2004277298
(71) Applicant: Shiseido Company, Limited, Chuo-ku Tokyo 104-8010 (JP)
(72) Inventor: UMISHIO, Kenichi, SHISEIDO RESEARCH CENTER, Yokohama-shi, Kanagawa 224-8558 (JP); MAEDA, Kazuhisa, SHISEIDO RESEARCH CENTER, Yokohama-shi, Kanagawa 224-8558 (JP); KOBAYASHI, Koji, SHISEIDO RESEARCH CENTER, Yokohama-shi, Kanagawa 224-8558 (JP)
(74) Representative: Santarelli
(86) International application number: PCT/JP2005/017434
(87) International publication number: WO 2006/033372

(57) **Abstract**

A whitening agent containing a solvent extract of a plant *Elephantopus mollis H. B. & K.* belonging to *Compositae* having a superior whitening action and having a superior effect in lightening and whitening pigmentation after suntans, senile lentigo, freckles, melasma, is provided.

## Description

### TECHNICAL FIELD

The present invention relates to a whitening agent, more specifically relates to a whitening agent effective for the prevention and improvement of pigmentation after suntan, senile lentigo, freckles, and melasma and the like and having a superior effect in skin whitening.

### BACKGROUND ART

While there are some unclear points regarding the mechanism for formation of senile lentigo in skin, in general, it is believed that hormonal abnormalities and irritation by ultraviolet rays from sunlight cause the formation of melanin pigment, which abnormally settle in skin. This melanin pigment causing coloring of skin is formed in the melanosomes in melanocytes between the epidermis and dermis. The formed melanin diffuses to the adjoining cells due to an osmotic action. The biochemical reaction in the melanocytes is believed to be as follows:
That is, tyrosin, an essential amino acid becomes dopaquinone by the action of the enzyme tyrosinase, and then the dopaquinone transforms through a red pigment and a colorless pigment to a black melanin by an enzymatic or nonenzymatic oxidizing action. This is the process for the production of melanin pigment.
In the past, various plant-derived extracts have been developed as a whitening agent suppressing the production of melanin pigment with the expectation of safety and reduced irritation to skin (e.g., see Japanese Patent Publication (A) No. 8-310939, Japanese Patent Publication (A) No. 7-89843 and Japanese Patent Publication (A) No. 9-30954).

### DISCLOSURE OF THE INVENTION

As explained above, in the plant-derived whitening agents, it is desired to find new plants exhibiting new whitening effects. Accordingly, the object of the present invention is to provide a new plant-derived whitening agent for dealing with the above-mentioned conventional situation.

In accordance with the present invention, there is provided a whitening agent comprising a solvent extract of the plant *Elephantopus mollis H. B. & K.* belonging to *Compositae.*

In accordance with the present invention, there is also provided an external skin preparation composition containing a solvent extract of the above plant, *Elephantopus mollis H. B. & K.* belonging to *Compositae,* and an external skin preparation vehicle.

The whitening agent of the present invention has a superior whitening action and has a superior effect in whitening the pigmentation after suntan, senile lentigo, freckles, melasma, and so forth.

### BEST MODE FOR CARRYING THE INVENTION

The inventors, in view of the above situation, engaged in repeated in-depth studies and, as a result, found that a specific *Compositae* plant has a whitening action, whereby the present invention has been completed.

In this description and claims, singular forms shall include plural forms so long as it is not clear otherwise from the context.

The plant *Elephantopus mollis H. B. & K.* belonging to *Compositae* used in the present invention is over 1 meter in height and inhabits in the tropics of East Asia and on the somewhat wet slopes along rivers in Ogasawara and Okinawa. The Japanese name is *Shirobanaigakouzorina.*

The extract of *Elephantopus mollis H. B. & K.* used in the present invention is obtained by immersing the leaves, stem, flowers, bark, seeds or fruits of the plant or the plant, as a whole, together with an extraction solvent at a temperature of 10 to 90°C, preferably room temperature, or heating and refluxing, then filtering and concentrating the outcome; however an extract from leaves is particularly preferred. The extraction solvent used in the present invention can be any solvent, which is usually used for extraction. In particular, alcohols such as methanol, ethanol, 1,3-butylene glycol; water-containing alcohols; acetone, ethyl acetate, or other organic solvents may be used alone or in any combination thereof, but alcohols such as methanol, ethanol, 1,3-butylene glycol, or other alcohols are particularly preferred. In the case of an extraction solvent containing a large amount of water, extraction of active ingredients is suppressed; and, therefore, this is not preferred.

The amount of the whitening agent including the extract of *Elephantopus mollis H. B. & K.* in the external skin preparation composition according to the present invention is not particularly limited, but is, by dry weight, 0.0005 to 20.0 mass%, preferably 0.001 to 10.0 mass% in the total amount of the external preparation composition. If the amount is less than 0.0005 mass%, the effects of the present invention may not be sufficiently exhibited. Furthermore, if the amount is more than 20.0 mass%, sometimes formation into a preparation becomes difficult. Note that, even if the amount is not less than 10.0 mass%, further improvement in the effect cannot be recognized.

Furthermore, the external skin preparation composition of the present invention may suitably and optionally include, in addition to the extract of *Elephantopus mollis H. B. & K.,* as a vehicle, ingredients used for usual cosmetics or pharmaceuticals or other external skin preparations such as a humectant, antioxidant, oil ingredient, ultraviolet absorbent, surfactant, thickener, alcohol, powder ingredients, coloring material, water-based ingredient, water, various skin nutrients, etc. In addition, disodium edetate, trisodium edetate, sodium citrate, sodium polyphosphate, sodium metaphosphate, gluconic acid, and other chelates, caffeine, tannin, verapamil, tranexamic acid and the derivatives, licorice extract, glabridin, hot water extracts of fruit of the firethorn, various herbs, tocopherol acetate, glycyrrhizic acid and the derivatives or the salts thereof or other medicines, vitamin C, magnesium ascorbyl phosphate, ascorbyl glucoside, arbutin, kojic acid, and other whitening agents, glucose, fructose, mannose, sucrose, trehalose, or other saccharides etc. may be suitably included.

The external skin preparation composition of the present invention may be used for any of an ointment, cream, milk lotion, lotion, pack, bath agent, or other conventional external skin preparation composition. The form of the preparation is not particularly limited.

### Examples

The present invention will now be explained in further detail below by giving examples; however the present invention is not limited by these examples in any way. Note that the amounts blended are shown by mass% unless specially indicated.

First, the test methods and results relating to the effect of suppression in melanin production by the plant extract of the present invention will be explained.

### 1. Preparation of Samples

### (A) Liquid extract with 1,3-butylene glycol

### (1) 1,3-Butylene glycol extract of Elephantopus mollis H. B. & K.

5.0 g of leaves of *Elephantopus mollis H. B. & K.* were used, and immersed in 50 mL of 1,3-butylene glycol at room temperature for seven days, then filtered to obtain a 1,3-butylene glycol extract. The solids concentration thereof was 0.16%.

### (2) 1,3-Butylene glycol extract of Elephantopus scaber L.

5.0 g of leaves of *Elephantopus scaber L.* were used, and immersed in 50 mL of 1,3-butylene glycol at room temperature for seven days, then filtered to obtain a 1,3-butylene glycol (1,3-BG) extract. The solids concentration was 0.13%.

### (B) Ethanol Extracts

### (1) Ethanol extract of Elephantopus mollis H. B. & K.

5.0 g of leaves of *Elephantopus mollis H. B. & K.* were used, and immersed in 50 mL of ethanol at room temperature for seven days, then the solvent was distilled off to thereby obtain an ethanol extract in an amount of 225 mg.

### (2) Ethanol extract of Elephantopus scaber L.

5.0 g of leaves of *Elephantopus scaber L.* were used, and immersed in 50 mL of ethanol at room temperature for seven days, then the solvent was distilled off to thereby obtain an ethanol extract in an amount of 213 mg.

These extracts were used for the following tests.

### 2. Test Method of Effect of Suppression of Melanin Production and Results

The 1,3-butylene glycol extracts and the ethanol extracts obtained above were used as the test samples to determine and evaluate the effect of suppression in the melanin production.

Mouse B16 melanoma cells were used. A medium composed of Eagle MEM containing fetal bovine serum (FBS, 10%) and a melanocyte stimulating hormone (αMSH, 10 ng/mL) was used as the test medium. The cells were incubated and propagated in a CO₂ incubator in a 75 cm² flask using an Eagle MEM medium containing FBS (10%). The cells were disassociated with a trypsin solution, an Eagle MEM medium containing FBS (10%) was added, then the mixture was centrifuged at 1,100 rpm in order to collect the cells. Next, a dish (100Φ × 20 mm) was inoculated with 300,000 cells. These were incubated in an Eagle MEM medium containing 5 mL of FBS (10%) for 1 day. After exchanging the medium to the test medium, a test sample was added by two concentrations (as solids, 2.5 and 5 ppm), then incubated for 3 days. The following method was used to measure the amount of melanin per cell.

Furthermore, the 1,3-butylene glycol extracts and the ethanol extracts from the *Elephantopus mollis H. B. & K.* having the strong activities were similarly tested at lower test sample concentrations (as solids, 0.3125, 0.625, and 1.25 ppm).

The cells were disassociated with 5 mL of trypsin solution and transferred to a 15 mL centrifugation tube. 5 mL of PBS was added to the dish, then the mixture was transferred to the same centrifugation tube. The number of cells was measured by a Coulter Z1, then the mixture was centrifuged at 1,100 rpm to collect the cells. These were air dried, then 2M sodium hydroxide solution was added to 100 µL/10,000 cells and the mixture was warmed at 60°C for 3 minutes. This was stirred to dissolve the melanin, then the degree of coloring was compared by the naked eye. Furthermore, if necessary, a control (1,3-butylene glycol extract or ethanol extract not added) was diluted with a 2M sodium hydroxide solution to an absorbance at 500 nm of 0.100. Different melanin solutions diluted to the same strengths were measured for absorbance at 500 nm, as an indicator of the degree of coloring.

The results in the case of use of a 1,3-butylene glycol extract are shown in Table I, while the results in the case of use of an ethanol extract are shown in Table II.

**Table I**

| Sample | Test concentration (ppm) | Naked eye judgment on coloring by comparison with control | 500 nm absorbance |
|---|---|---|---|
| *Elephantopus mollis H. B. & K.* | 0.3125 | Slightly lightened | 0.092 |
| | 0.625 | Slightly lightened | 0.099 |
| | 1.25 | Moderately lightened | 0.079 |
| | 2.5 | Remarkably lightened (almost colorless) | Not measured |
| | 5 | Remarkably lightened (almost colorless) | Not measured |
| *Elephantopus scaber L.* | 2.5 | No change | 0.103 |
| | 5 | Moderately lightened | 0.087 |

**Table II**

| Sample | Test concentration (ppm) | Naked eye judgment on coloring by comparison with control | 500 nm absorbance |
|---|---|---|---|
| *Elephantopus mollis H. B. & K*. | 0.3125 | Slightly lightened | 0.100 |
| | 0.625 | Slightly lightened | 0.091 |
| | 1.25 | Moderately lightened | 0.083 |
| | 2.5 | Remarkably lightened (almost colorless) | Not measured |
| | 5 | Remarkably lightened (almost colorless) | Not measured |
| *Elephantopus scaber L.* | 2.5 | Slightly lightened | 0.100 |
| | 5 | Slightly lightened | 0.095 |

From the results shown in Table I and Table II, it is clear that, in the present test method believed to be closed to in vivo conditions, the 1,3-butylene glycol extract and the ethanol extract from *Elephantopus mollis H. B. & K.* have higher effects at the same concentrations, compared with the 1,3-butylene glycol extract and the ethanol extract from *Elephantopus scaber L.*

Examples of formulation of external skin preparations using the whitening agent of the present invention in various forms will now be explained as Working Examples.

### Example 1: Cream

### (Formulation)

| Ingredients | mass% |
|---|---|
| Stearic acid | 5.0 |
| Stearyl alcohol | 4.0 |
| Isopropyl myristate | 18.0 |
| Glycerol monostearate | 3.0 |
| Propylene glycol | 10.0 |
| Methanol extract of *Elephantopus mollis H. B. & K.* | 0.01 |
| Potassium hydroxide | 0.2 |
| Sodium bisulfite | 0.01 |
| Preservative | q.s. |
| Perfume | q.s. |
| Ion exchanged water | Balance |

### (Method of Preparation)

The propylene glycol, methanol extract of *Elephantopus mollis H. B. & K*., and potassium hydroxide were added to the ion exchanged water and dissolved. The mixture was heated and kept at 70°C (aqueous phase). The other ingredients were mixed, heated to dissolve, and kept at 70°C (oil phase). The oil phase was gradually added to the aqueous phase. After the entire amount was added, the mixture was kept at the temperature to cause a reaction. Thereafter, the mixture was uniformly emulsified by a homomixer and stirred well and cooled down to 30°C.

### Example 2: Cream

### (Formulation)

| Ingredients | mass% |
|---|---|
| Stearic acid | 2.0 |
| Stearyl alcohol | 7.0 |
| Hydrogenated lanolin | 2.0 |
| Squalane | 5.0 |
| 2-Octyldodecyl alcohol | 6.0 |
| Polyoxyethylene (25) cetyl ether | 3.0 |
| Glycerol monostearate | 2.0 |
| Propylene glycol | 5.0 |
| Ethanol extract of *Elephantopus mollis H. B. & K.* | 0.05 |
| Sodium bisulfite | 0.03 |
| Ethyl paraben | 0.3 |
| Perfume | q.s. |
| Ion exchanged water | Balance |

### (Method of Preparation)

The propylene glycol was added to the ion exchanged water, then the mixture was heated and kept at 70°C (aqueous phase). The other ingredients were mixed, heated to melt, then kept at 70°C (oil phase). The oil phase was added to the aqueous phase and preemulsified. The mixture was uniformly emulsified by a homomixer, then stirred well and cooled down to 30°C.

### Example 3: Cream

### (Formulation)

| Ingredients | mass% |
|---|---|
| Solid paraffin | 5.0 |
| Beeswax | 10.0 |
| Vaseline | 15.0 |
| Liquid paraffin | 41.0 |
| Glycerol monostearate | 2.0 |
| Polyoxyethylene (20) sorbitan monolaurate | 2.0 |
| Soap powder | 0.1 |
| Borax | 0.2 |
| Acetone extract of *Elephantopus mollis H. B. & K.* | 0.05 |
| Ethanol extract of *Elephantopus mollis H. B. & K.* | 0.05 |
| Sodium bisulfite | 0.03 |
| Ethyl paraben | 0.3 |
| Perfume | q.s. |
| Ion exchanged water | Balance |

### (Method of Preparation)

The soap powder and borate were added to the ion exchanged water, then the mixture was heated to dissolve and kept at 70°C (aqueous phase). The other ingredients were mixed, heated to melt, then kept at 70°C (oil phase). The oil phase was gradually added to the aqueous phase while stirring for reaction. After the end of the reaction, the mixture was uniformly emulsified by a homomixer, then, after being emulsified, stirred well and cooled down to 30°C.

### Example 4: Milky Lotion

### (Formulation)

| Ingredients | mass% |
|---|---|
| Stearic acid | 2.5 |
| Cetyl alcohol | 1.5 |
| Vaseline | 5.0 |
| Liquid paraffin | 10.0 |
| Polyoxyethylene (10) monooleate | 2.0 |
| Polyethylene glycol 1500 | 3.0 |
| Triethanol amine | 1.0 |
| Carboxyvinylpolymer | 0.05 |
| Ethyl acetate extract of *Elephantopus mollis H. B. & K.* | 0.01 |
| Sodium bisulfite | 0.01 |
| Ethyl paraben | 0.3 |
| Perfume | q.s. |
| Ion exchanged water | Balance |

### (Method of Preparation)

The carboxyvinylpolymer was dissolved in a small amount of ion exchanged water (A-phase). The polyethylene glycol 1500 and triethanol amine were added to the remaining ion exchanged water, then heated to dissolve and kept at 70°C (aqueous phase). The other ingredients were mixed, heated to melt, then kept at 70°C (oil phase). The oil phase was added to the aqueous phase, the mixture was preemulsified, then the A-phase was added. The mixture was uniformly emulsified by a homomixer, then, after being emulsified, stirred well and cooled down to 30°C.

### Example 5: Milky Lotion

### (Formulation)

| Ingredients | mass% |
|---|---|
| Microcrystalline wax | 1.0 |
| Beeswax | 2.0 |
| Lanolin | 20.0 |
| Liquid paraffin | 10.0 |
| Squalane | 5.0 |
| Sorbitan sesquiolate | 4.0 |
| Polyoxyethylene (20) sorbitan monooleate | 1.0 |
| Propylene glycol | 7.0 |
| Acetone extract of *Elephantopus mollis H. B. & K.* | 10.0 |
| Sodium bisulfite | 0.01 |
| Ethyl paraben | 0.3 |
| Perfume | q.s. |
| Ion exchanged water | Balance |

### (Method of Preparation)

Propylene glycol was added to the ion exchanged water, then the mixture was heated and kept at 70°C (aqueous phase). The other ingredients were mixed, then heated to melt and kept at 70°C (oil phase). While stirring the oil phase, the aqueous phase was gradually added, then the mixture was uniformly emulsified by a homomixer. After the emulsification, it was stirred well and cooled down to 30°C.

### Example 6: Gel

### (Formulation)

| Ingredients | mass% |
|---|---|
| 95% ethyl alcohol | 10.0 |
| Dipropylene glycol | 15.0 |
| Polyoxyethylene (50) oleyl alcohol ether | 2.0 |
| Carboxyvinylpolymer | 1.0 |
| Sodium hydroxide | 0.15 |
| L-arginine | 0.1 |
| Ethanol extract of *Elephantopus mollis H. B. & K.* | 7.0 |
| Sodium 2-hydroxy-4-methoxybenzophenone sulfonate | 0.05 |
| Trisodium ethylenediamine tetraacetate dihydrate | 0.05 |
| Methyl paraben | 0.2 |
| Perfume | q.s. |
| Ion exchanged water | Balance |

### (Method of Preparation)

The carboxyvinyl polymer was uniformly dissolved in the ion exchanged water. On the other hand, the ethanol extract of *Elephantopus mollis H. B. & K*. and the polyoxyethylene (50 mol) oleyl alcohol ether were dissolved in the 95% ethanol, then the mixture was added to the aqueous phase. Next, the other ingredients were added, then the mixture was neutralized and thickened with the sodium hydroxide and L-arginine.

### Example 7: Essence

### (Formulation)

| Ingredients | mass% |
|---|---|
| (A-phase) | |
| Ethyl alcohol (95%) | 10.0 |
| Polyoxyethylene (20) octyldodecyl ether | 1.0 |
| Pantothenyl ethylether | 0.1 |
| 1,3-Butylene glycol extract of *Elephantopus mollis H. B. & K.* (solids concentration 0.16%) | 1.5 |
| Methyl paraben | 0.15 |

| (B-phase) | |
|---|---|
| Potassium hydroxide | 0.1 |

| (C-phase) | |
|---|---|
| Glycerol | 5.0 |
| Dipropylene glycol | 10.0 |
| Sodium bisulfite | 0.03 |
| Carboxyvinylpolymer | 0.2 |
| Purified water | Balance |

### (Method of Preparation)

The A-phase and the C-phase were separately and uniformly dissolved, then the A-phase was added to the C-phase to solubilize it. Next, the B-phase was added, then the mixture was filled in a container.

### Example 8: Pack

### (Formulation)

| Ingredients | mass% |
|---|---|
| (A-phase) | |
| Dipropylene glycol | 5.0 |
| Polyoxyethylene (60 mol) hydrogenated castor oil | 5.0 |

| (B-phase) | |
|---|---|
| Methanol extract of *Elephantopus mollis H. B. & K.* | 0.01 |
| Olive oil | 5.0 |
| Tocopheryl acetate | 0.2 |
| Ethyl paraben | 0.2 |
| Perfume | 0.2 |

| (C-phase) | |
|---|---|
| Sodium bisulfite | 0.03 |
| Polyvinyl alcohol (saponification degree 90, polymerization degree 2,000) | 13.0 |
| Ethanol | 7.0 |
| Purified water | Balance |

### (Method of Preparation)

The A-phase, B-phase and C-phase were separately and uniformly dissolved, then the B-phase was added to the A-phase to solubilize it. Next, this mixture was added to the C-phase, then filled in a container.

### Example 9: Solid Foundation

### (Formulation)

| Ingredients | mass% |
|---|---|
| Talc | 43.1 |
| Kaolin | 15.0 |
| Sericite | 10.0 |
| Zinc white | 7.0 |
| Titanium dioxide | 3.8 |
| Yellow iron oxide | 2.9 |
| Black iron oxide | 0.2 |
| Squalane | 8.0 |
| Isostearic acid | 4.0 |
| Polyoxyethylene sorbitan monooleate | 3.0 |
| Isocetyl octanoate | 2.0 |
| Ethanol extract of *Elephantopus mollis H. B. & K.* | 1.0 |
| Preservative | q.s. |
| Perfume | q.s. |

### (Method of Preparation)

The powder ingredients from the talc to black iron oxide were sufficiently mixed by a blender, then the oil ingredients from the squalan to isocetyl octanoate, the ethanol extract of *Elephantopus mollis H. B. & K*., the preservative and the perfume were added and mixed well, then filled in a container to shape it.

### Example 10: Emulsion Type Foundation (Cream Type)

### (Formulation)

| Ingredients | mass% |
|---|---|
| (Powder part) | |
| Titanium dioxide | 10.3 |
| Sericite | 5.4 |
| Kaolin | 3.0 |
| Yellow iron oxide | 0.8 |
| Red iron oxide | 0.3 |
| Black iron oxide | 0.2 |

| (Oil phase) | |
|---|---|
| Decamethyl cyclopentasiloxane | 11.5 |
| Liquid paraffin | 4.5 |
| Polyoxyethylene modified Polydimethylsiloxane | 4.0 |

| (Aqueous phase) | |
|---|---|
| Purified water | 50.0 |
| 1,3-butylene glycol | 4.5 |
| Ethanol extract of *Elephantopus mollis H. B. & K.* | 1.5 |
| Sorbitan sesquioleate | 3.0 |
| Preservative | q.s. |
| Perfume | q.s. |

### (Method of Preparation)

The aqueous phase was heated and stirred, then the sufficiently mixed and pulverized powder part was added and processed by a homomixer. Furthermore, the heated and mixed oil phase was added and processed by a homomixer, then the mixture was stirred, the perfume was added, and the resultant mixture was cooled down to room temperature.

## Claims

1. A whitening agent comprising a solvent extract of a plant *Elephantopus mollis H. B. & K.* belonging to a family *Compositae.*

2. A whitening agent as claimed in claim 1, wherein said solvent extract is obtained by extraction of leaves, the stems, flowers, bark, seeds or fruits of said plant belonging to *Compositae* extracted with an extraction solvent.

3. A whitening agent as claimed in claim 2 wherein said extraction solvent is an alcohol.

4. An external skin preparation composition comprised of a solvent extract of an *Elephantopus mollis H. B. & K.* belonging to a family according to any one of claims 1 to 3 and an external skin preparation vehicle.

5. An external skin preparation composition as claimed in claim 4, wherein the amount of the whitening agent is 0.0005 to 20.0 mass% in terms of a dried extract based upon the weight of the whitening external skin preparation composition.
